# EUROPEAN PATENT SPECIFICATION

(11) **EP 2 248 799 B1**
(45) Date of publication and mention of the grant of the patent: **20.05.2015**
(21) Application number: 10174144.5
(22) Date of filing: 19.12.2002
(51) Int. Cl.: C07C 269/04, C07C 271/12, C07C 69/757, C07C 33/46

(54) **PROCESS FOR PREPARING 2-(SUBSTITUTED PHENYL)-2-HYDROXY-ETHYL CARBAMATES**
VERFAHREN ZUR HERSTELLUNG VON 2-(SUBSTITUIERTE PHENYL)-2-HYDROXY-ETHYL-CARBAMATE
PROCÉDÉ DE PRÉPARATION DE PHÉNYL (-2-SUBSTITUÉ)-2-HYDROXY-ÉTHYL-CARBAMATES

(30) Priority: 11.10.2002 US 418084 P; 21.12.2001 EP 01205097
(43) Date of publication of application: 10.11.2010
(62) Divisional of application: 02793127.8
(73) Proprietor: SK Biopharmaceuticals Co., LTD, Jongro-gu Seoul 110-110 (KR)
(72) Inventor: Otten, Thomas, 8052 Zurich (CH); Rey, Max, Di, Deceased (CH); Korey, Daniel, Spring House PA Pennsylvania 19477-0776 (US)
(74) Representative: Warner, James Alexander

(56) References cited:
- EP-A- 0 459 455
- WO-A-01/40168
- WO-A-97/26241
- US-A- 5 504 108

## Description

### Brief description of the invention

The present invention relates to a new process for preparing (S) - (+) - 2- (substituted phenyl) - 2- hydroxy-ethyl carbamates and to novel intermediates used in this process. It further relates to polymorphic forms of said carbamates.

### Background of the Invention

2- (Substituted phenyl) - 2- hydroxy-ethyl carbamates and their isomers have been described in US-5,698,588 and in US-5,854,283, which corresponds to WO-97/26241, as compounds useful for treating disorders of the central nervous system in particular convulsions, epilepsy, stroke, muscle spasms, neuropathic pain and migraine. These compounds have an asymmetric carbon atom at the benzylic position, which is the aliphatic carbon atom adjacent to the phenyl ring. This asymmetric carbon atom preferably is in an S-configuration. A preferred compound among these carbamates is (S) - (+) - 2- (2-chlorophenyl) - 2- hydroxy-ethyl carbamate, which compound is also referred to as RWJ-333369. This and similar optically pure forms of carbamates of halogenated 2-phenyl-1,2-ethanediol have been described in US-6,127,412 and US-6,103,759.

These compounds are prepared by reacting the appropriately substituted phenyl-1,2-ethanediol with dimethylcarbonate thus yielding the corresponding phenyl-1,2-ethanediol carbonate which subsequently was reacted with a suitable amine which yielded two regioisomeric forms of the resulting carbamates which had to be separated by column chromatography. Stereoisomerically pure compounds were prepared similarly, starting from the enantiomerically pure starting material. This process results in a mixture of two regioisomeric products, one of which needs to be removed, which has a negative impact on the yield. Both regioisomeric end products are separated by chromatography which is a cumbersome procedure, especially in large scale production.

It is an object of this invention to provide a process that not only avoids this separation step and concomitant loss of undesired regioisomer, but also results in end product in high yield and purity, in particular in high enantiomeric purity. A further object is to provide a process that can be scaled up to large production batch sizes, to provide a process that uses starting materials that are easy to produce or are commercially available. It is still a further object to provide a process that is cost-effective and does not give rise to side products that are hazardous or difficult to remove. It is another object of the present invention to provide a process that has a limited number of purification steps, in particular a process wherein some or all of the intermediates need no purification. A still further object is to provide a process that avoids excess quantities of starting materials and solvents.

These objects are attained by the process of the present invention.

Drug substances, also referred to as active pharmaceutical ingredients, can occur as polymorphic forms. In almost all instances, a particular polymorphic form has different properties compared to the active ingredient not occurring in a specific polymorphic form or compared to another polymorphic form of an active ingredient. Properties may differ as far as solubility, stability, flowability, tractability, compressibility, etc., are concerned. These differences in turn are known to affect the properties of the formulations made of such active ingredients as well as the final or finished dosage forms thereof. Authorities responsible for drug approval in many countries require the complete characterization of the active ingredient used in each drug product, including the identification and control of polymorphic forms. If present in the finished product, drug approving authorities require the manufacturer of the active substance, in the least, to control its synthetic processes such that the percentages of the various respective polymorphic forms, when present, are consistent among batches and within the drug substance's approved specifications. Left uncontrolled in synthetic processes, the percentage of a given polymorph can fluctuate therefore having an effect of the properties of the active substance and the finished drug product such that it no longer meets the specifications of the drug approval. Therefore a robust control is required of the synthetic process specifications and of the end product, in particular as far as the presence of consistent amounts of polymorphic forms is concerned.

Many active substances do not show polymorphism and the presence of polymorphic forms of a new chemical active ingredient is not readily predictable. Variations in the process parameters can lead to varying degrees of the presence of polymorphic forms in end products, which, as explained above is undesirable. Therefore the knowledge of the presence of polymorphic forms and their characterization is a highly desirable goal to achieve.

. Quite unexpectedly it has now been found that (S) - (+) - 2- (substituted phenyl) - 2-hydroxy-ethyl carbamates occur as polymorphic forms that can be isolated and characterized. In an additional aspect, this invention provides practical and reproducible procedures to prepare these polymorphic forms.

### Summary of the Invention

The present invention relates to a process of preparing a compound of formula (I): 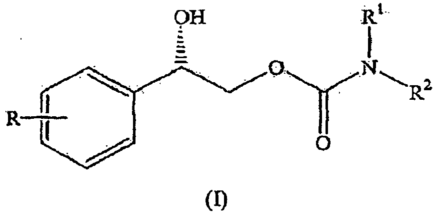 wherein:
R is halo;
R¹ and R² independently are hydrogen or C₁₋₄alkyl, optionally substituted with phenyl or substituted phenyl, wherein substituted phenyl has substituents selected from halogen, C₁₋₄alkyl, C₁₋₄alkyloxy, amino, nitro and cyano;
   characterized by
   (a) reducing an ester of formula: 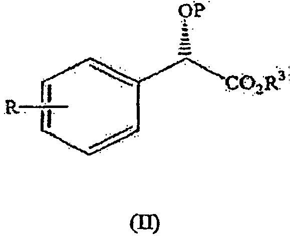 wherein:
      P is an appropriate alcohol-protecting group;
      R³ is C₁₋₄alkyl;
      with an appropriate ester-to-alcohol reducing agent, thus obtaining an alcohol of formula: 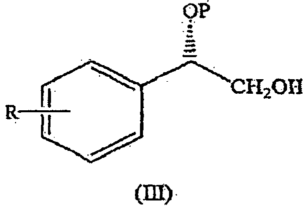
   (b) reacting the alcohol of formula (III) with a carbonyl compound of formula 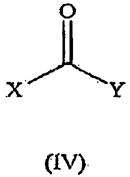 wherein X and Y are appropriate leaving groups; and subsequently with an amine of formula 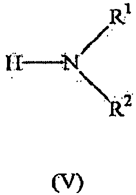 thus obtaining a compound of formula: 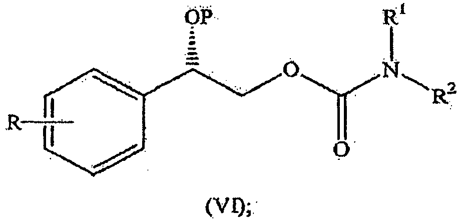
   (c) removing the protecting group P thus obtaining a compound of formula (I).

In some instances one or more of the substituents R¹, R² and R³ may have asymmetric carbon atoms and hence may cause the compounds of formula (I) to occur in stereoisomeric forms. Such stereoisomeric forms are intended to be embraced within the scope of the present invention.

Preferred compounds of formula (I) are those wherein R is 2-chloro, R¹ and R² are hydrogen.

The group P is an appropriate alcohol protecting group. Preferred groups P are of the ether type. A particularly preferred protecting group P is 2-(2-methoxy)propyl.

In a preferred execution of the process the carbonyl compound of formula (IV) is selected from 1,1'-carbonyl-diimidazole and phenyl chloroformiate.

A particular execution of the process is that wherein R³ is methyl.

Another particular execution of the process is that wherein the appropriate ester-to-alcohol reducing agent is a metal hydride or a complex metal hydride.

In a further aspect the invention relates to a compound of formula 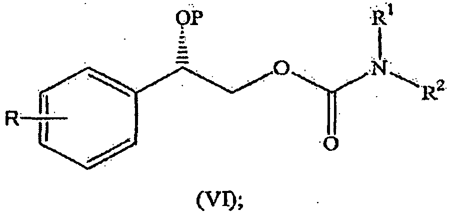 wherein R, R¹ and R² are as defined in claims 1 or 2 and P is an appropriate hydroxy-protecting group.

The invention further provides a process preparing a compound of formula (I) Characterised by process steps (d) and (e) as outlined hereinafter and by process steps (a), (b) and (c) as outlined above.

### Detailed Description of the Invention

Subject of the present invention is a process for preparing compounds of formula (I), as outlined above, and the intermediate of formula (VI) as represented and defined hereinabove.

Preferred are those compounds and intermediates as defined herein wherein R is 2-chloro, R¹ and R² are hydrogen. The compound of formula (I) wherein the substituents have the latter meanings is also referred to as 'RWJ-333369' and can be represented by the structural formula:

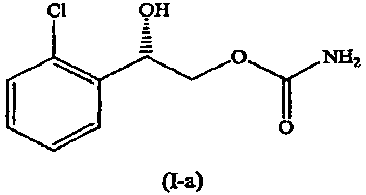

The term "halogen" refers to fluoro, chloro, bromo and iodo.

"C₁₋₄alkyl" defines straight and branched chain saturated hydrocarbon radicals having from 1 to 4 carbon atoms such as methyl, ethyl, 1-propyl, 2-propyl, 1-butyl, 2-butyl, 2-methyl-1-propyl, 2-methyl-2-propyl.

"C₁₋₄alkyloxy" defines C₁₋₄alkyl radicals linked to an oxygen atom such as methoxy, ethoxy, 1-propoxy, 2-propoxy, 1-butoxy, 2-butoxy and the like.

"Substituted phenyl" is phenyl being substituted with the substituents outlined above. Preferably, substituted phenyl has 1, 2 or 3 substituents. A preferred substituent is halogen, more preferably chloro.

P, as mentioned above, is an appropriate hydroxy protecting group. Preferably it should be selected such that it is stable in the reduction procedure of (II) to (III) as well as the subsequent reaction step from (III) to (VI). The preferred reduction procedure being with complex hydrides as outlined hereinafter, the group P should be stable towards these complex hydrides and the reaction products therefrom. The reduction with complex hydrides requires basic conditions and hence the group P should not be cleaved in these basic conditions. The group P should preferably be removable under acidic conditions which should be such that the carbamate function is not split. Particularly preferred are protecting groups P that are removable at a pH which is about 1 or slightly higher.

Particularly useful are groups P of the ether type. As examples of P there may be mentioned:
methoxymethyl ethers (MOM): which can be prepared from methoxymethylchloride or formaldehyde dimethylacetal;
tetrahydropyran ethers (THP ethers) prepared from dihydropyran;
tetrahydrothioypyranyl ethers from dihydrothiopyran;
chloro substituted tetrahydrofuranyl ethers from 2-chlorotetrahydrofuran;
tetrahydrothiofuranyl ethers from dihydrothiofuran;
1-ethoxyethyl ethers from ethylvinyl ethers or 1-ethoxyethyl chloride
1-methyl-1-methoxyethyl ethers from methylvinyl ethers, which are of particular interest;
triphenyl ethers and appropriate derivatives thereof which can be prepared from the corresponding chlorides;
benzyl ethers and appropriate derivatives thereof which can be prepared from the corresponding bromides or iodides;
4-methoxytetrahydropyranyl ethers from 5,6-dihydro-4-methoxy-2H-pyran;
4-methoxytetrahydrothiopyranyl ethers from 5,6-Dihydro-4-methoxy-2H-thiopyran.

It is furthermore advantageous to use such groups P which do not lead to a mixture of diastereomers, i.e. P groups without an asymmetric center. Under certain circumstances, e.g. depending upon the nature of the reducing agent, particular silyl ethers can be used as appropriate groups P, in particular t.butyldimethylsilyl (TBDMS), triisopropylsilyl, tribenzylsilyl, and the like.

### Step (a): preparation of 2-(substituted phenyl)-2-(protected hydroxy)-ethanol (III)

According to the first step of the process of this invention, the ester (II) is reduced to the corresponding alcohol of formula (III) using an appropriate ester-to-alcohol reducing agent. The latter may be a metal hydride or a complex metal hydride such as lithium aluminium hydride or derivatives thereof.

Particular reduction agents for this reaction are silane agents such as trialkylsilanes, dialkylsilanes, trialkoxysilanes and preferably poly methylhydrogensiloxane ('PMHS') in the presence of a suitable catalyst. The latter in particular are transition metal halogenides or carboxylates, and preferably the latter is a zinc carboxylate, such as zinc hexanoate or a derivative thereof, more preferably zinc 2-ethylhexanoate, in the presence of a metal hydride such as an alkali metal or earth alkaline metal hydride, or aluminium hydride, e.g. lithium, sodium, potasiwn, calcium hydride, or a complex hydride such as a borohydride or aluminium hydride, in particular an alkali metal borohydride or aluminiumhydride, e.g. lithium, sodium or potassium borohydride or aluminium hydride. A combination of zinc 2-ethylhexanoate and sodium borohydride is most preferably used as the catalyst mixture. These and similar reduction agents are described in Patent Application WO 96/12694 (1995) and in J. Ulman, The Alembic, 1999, 59, 1 ff.

The reaction of this process step is conducted in a suitable solvent, e.g. an ether or polyether, or a hydrocarbon, in particular an aromatic hydrocarbon. Specific examples of suitable solvents comprise diethyl ether, dipropyl ether, diisopropyl ether, dibutyl ether, diglyme and toluene.

The reduction agent can be formed by stirring a mixture of the agents ERS A and ERS B in a separate reaction vessel (ERS = Ester Reduction System), commercially available from Rohm & Haas). ERS A is a solution of NaBH₄ in tetraglyme, whereas ERS B is a solution of Zn(carboxylate)2*H₂O, in particular of Zn(2-ethylhexanoate)₂ *H₂O, in tetraglyme. ERS A and ERS B are preferably mixed at higher temperature, e.g. at a temperature in the rage of 50 to 90°C, in particular at 70°C for a period of several minutes, e.g. for 30 min. Subsequently the ERS A and ERS B mixture is added to the ester (II) and ERS C is added. ERS C is poly methylhydrogensiloxane.

The reducing agent can also be generated *in situ* in the reaction vessel, which is particularly attractive. In this variant, intermediate (II) is dissolved in a suitable solvent, preferably in toluene at increased temperature of 80-90°C. ERS B is added first in one portion, followed by the addition of ERS A also in one portion. Then immediately ERS C is added within one hour while controlling the temperature and keeping it at higher temperature, in particular around 95°C.

The starting mandelic acid ester (II) is prepared as described hereinafter and can be isolated and optionally purified and used as such in the reduction step. The ester (II) can also be kept dissolved in the solvent in which it was prepared and used as such in the reduction step.

Preferably the substituted mandelic acid ester (II) is a methyl ester.

In a particular execution of this process step the starting ester (II) is either used dissolved in the solvent of the previous reaction step, or it can also be solved in a suitable solvent, such as for example an ether, e.g. in di-n-butylether, or preferably in toluene. The reducing agent, prepared as above, is added in one portion to the solution, followed by the addition of ERS C within one hour. The reaction mixture optionally can be heated to 90°C prior to addition of ERS C. Then the temperature is increased to 90°C and kept at that temperature until an in-process-control shows a conversion of at least 99%. The reaction time is around 1 h. After complete reduction the mixture is cooled to 15 to 20°C and hydrolyzed carefully first with methanol and subsequently with aqueous alkali metal hydroxide solution, which preferably is a NaOH solution in slight excess (e.g. 1.3 mol-eq., using a 30% solution).

Subsequently, the mixture is refluxed for about 1 h at about 50°C and the layers are separated at room temperature. The organic layer is washed with water and with saturated aqueous NaCl at room temperature.

The quantity of ERS C is in the range of 2 to 4, in particular in the range of 2.2 to 3 molar equivalents, preferably 2.3 molar equivalents of ERS C are used.

The reaction typically is complete after about 1 hour.

After completion of the reduction reaction, the excess of ERS C can be destroyed with a suitable ester, in particular with ethyl acetate. After addition of the said ester, the mixture is stirred for one hour and hydrolyzed at 90°C by addition of aqueous base solution, preferably a NaOH or a KOH-solution (e.g. of 33%) and further preferably with the use of methanol. Further work-up comprises the separation of the organic layer and washing with aqueous basic solution (e.g. KOH-solution of 33%) and with water.

The resulting methylester is an oil, and, if desired, can be distilled for purification.

### Step (b): preparation of 2-(substituted phenyl)-2-(protected hydroxy)-ethyl carbamate (VI)

In this step the alcohol of formula (III) is reacted with a carbonyl compound of formula 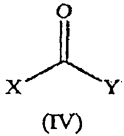 wherein X and Y are appropriate leaving groups. Preferably one of X or Y is more reactive than the other. X and Y can be halogen, in particular chloro or bromo, but preferably X and Y are imidazolyl groups. If X is halo, Y preferably is an aryloxy or alkoxy group. In the latter instance (V) is an alkyl or aryl halo formiate. Particular aryl groups in (IV) are phenyl or substituted phenyl, e.g. halophenyl, or C₁₋₄ alkyl. A preferred example of (IV) is phenyl chloroformiate or 1,1 '-carbonyl-diimidazole.

The reaction is conducted in a suitable solvent such as a hydrocarbon, in particular an aromatic hydrocarbon, e.g. toluene, or in an ether, e.g. THF. The reaction temperature depends upon the reactivity of the reagent (IV) but in general is room temperature or lower. In case N,N'-carbonyl diimidazole is used as reagent the reaction preferably is conducted at room temperature (i.e.. at about 25 °C).

The reaction typically is complete after less than 1 hour, e.g. after about ½ hour. The reaction product of this reaction is usually not isolated; it can be represented by the following formula: 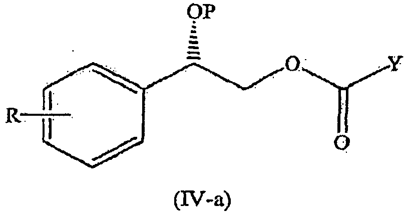 wherein Y is as defined hereinabove and in particular is an imidazolyl group, or an aryloxygroup, e.g. a phenoxy or substituted phenoxy group. The intermediates of formula (IV-a) are deemed novel and constitute an additional feature of the present invention. Preferred intermediates of formula (IV-a) are those wherein Y is imidazol-1-yl.

The product of the previous reaction, i.e. the intermediate of formula (IV-a), is usually not isolated and is reacted immediately with the amine of formula (V) as specified above. A preferred amine is ammonia, but it can also be an ammonium salt. In that instance the ammonia or an ammonium salt is in an aqueous medium and added to the solution of intermediate (IV-a) at room temperature.

Vigorous stirring is recommended when using (V) in aqueous media, because of the two phase system. The reaction is terminated after several hours, in particular after about 4 hrs. The organic phase is separated and the product can optionally be isolated and purified. The organic phase with product (VI) dissolved therein can also be used as such in the subsequent reaction step.

### Step c: preparation of 2-(substituted phenyl)-2-hydroxyethyl carbamate (I)

This reaction step involves the deprotection of the hydroxy function and depends on the nature of the group P.

Where P is 2-methoxy-2-propyl, removal is by addition of an appropriate acid, e.g. hydrochloric acid.

In a preferred execution, product (VI) is used dissolved in the solvent of the previous step. Water and concentrated hydrochloric acid are added while stirring. Already after a few minutes the reaction is almost completed and the end procuct (I) starts precipitating. The reaction mixture is stirred for a couple of hours, in particular for about 4 hours because in this way the end product can be filtered better. Subsequently the end product is filtered off and washed.

The end product (I) can be recrystallized from a suitable solvent such as an alcohol, e.g. methanol, preferably with addition of acidified water.

### Starting materials

The starting materials (II) may be obtained by a process which is characterized by:
(d) an esterification reaction of an appropriately substituted mandelic acid of formula: 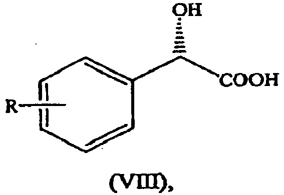 thus preparing the corresponding ester of formula 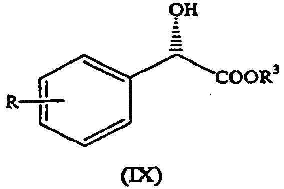 wherein in (VIII) and (IX) R and R³ are as defined above and R preferably is 2-chloro and/or R³ preferably is methyl; and
(e) treating the ester (IX) with a suitable reagent generating a hydroxy protecting group, thus preparing an intermediate of formula 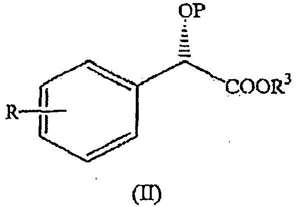 wherein R and P are as defined above.

The ester (IX) is reacted with an appropriate agent capable of introducing a hydroxy protecting group. A preferred agent is 2-methoxypropene.

Alternatively, the sequence of the above steps may be switched, i.e. the hydroxy protecting group may be introduced and subsequently the ester formed.

### Step (d): Preparation of substituted mandelic acid esters (IX)

According to this reaction step the starting acid (VIII) is reacted in the alcohol of which the ester (IX) is derived. Typically a C₁₋₄ alkanol is used, preferably methanol, thus yielding the corresponding C₁₋₄ alkyl esters or preferably the methyl ester of (VIII).

The reaction is conducted with an excess of a strong acid, preferably a hydrohalic acid such as HCl, in particular with 1-4 molar equivalents, preferably with 2.5 molar equivalents of concentrated HCl. The reaction also works with a catalytic amount of sulfuric acid or also with SOCl₂. In the latter instance the reaction is highly exothermic, requiring appropriate measures for controlling the temperature.

The reaction preferably is conducted at room temperature or slightly increased temperatures, preferably not higher than about 30°C. The reaction time usually is less than about 1 hour, e.g. about 30 min. The resulting ester (IX) typically is an oily compound which is used as such in the subsequent process steps. In a preferred execution, the ester (IX) is kept in the solvent in which it was made and is used further dissolved in this solvent.

### Step (e): Preparation of hydroxy-protected substituted mandelic acid esters (II).

The reaction conditions of this step depend on the nature of protecting group P.

In a preferred execution, P is 2-methoxy-1-propyl which is derived from 2-methoxypropene. The latter is dissolved in a suitable solvent, in particular the solvent in which the other reaction steps are conducted. This solution is added to a solution of intermediate (IX) in a suitable solvent which has been acidified, e.g. by addition of hydrochloric acid, in particular by adding gaseous hydrochloric acid over the solution. The pH should preferably be low, e.g. pH 1-2. The solvent of intermediate (IX) should preferably be the same as that wherein the 2-methoxypropene is dissolved and more preferably should be the same as the solvent used in the other reaction steps.

The reaction is complete in less than 1 hour, normally within half an hour.

The process according to the present invention yields the end product (I) in high yield and purity and can be scaled up to production size batches. A particular aspect of the current process is that it leaves the stereochemical integrity intact of the asymmetric center on the carbon atom bearing the hydroxy function, i.e. the present process shows neglectable or no racemisation.

The various intermediate products of the process, including the steps for preparing the starting materials, can be isolated and if desired further purified before further use in a next step. Alternatively, all the process steps, if desired also including the steps for preparing the starting materials, can be conducted in the same solvent, i.e. without isolation and optional purification of the intermediate products. In the latter instance it may be advantageous to distil off some of the solvent or to add some of it during one or more of the reaction steps. A suitable solvent for the one solvent execution of the process is an aromatic hydrocarbon, preferably toluene. It is also possible to conduct a certain number of steps in one solvent and the others in another.

Conducting the whole process in one solvent has the particular advantage that the process is much simpler and can be conducted more quickly without having to discard or recuperate solvents, which is advantageous economically as well as from an environmental aspect.

### Polymorphs

The compound of formula (I) wherein R is 2-chloro, R¹ and R² are hydrogen, said compound hereinafter being referred to as compound (I-a), occurs in different crystalline forms.

The crystalline forms of compound (I-a) may be prepared by an appropriate recrystallization of compound formula (I-a) from a suitable organic solvent. Depending upon the recrystallisation procedure either form A or form B can be obtained.

One crystalline form of compound (I-a) is referred to as 'form A' and is prepared by recrystallisation of compound (I-a) from a suitable solvent. The temperature in this recrystallisation procedure is kept below 60 °C, in particular below 50 °C. Suitable solvents are those wherein the compound of formula (I-a) dissolves at higher temperature and is relatively poorly soluble at lower temperature, e.g. at a temperature lower than 20 °C, or lower than 10 °C, or even lower than 0 °C, or -10 °C. Suitable solvents are the lower alkanols, i.e. the C₁₋₄alkanols and in particular methanol.

In one type of embodiment, the compound (I-a) is dissolved by heating or refluxing in methanol, or by heating in a lower alkanol. The temperature of the mixture should not allowed to exceed 60 °C. Subsequently, the solution is cooled, preferably slowly, e.g. by simply allowing the solution to cool. At a temperature of about 50 °C crystallization already starts. The mixture is stirred at room temperature for 15-20 hours whereupon acidified water is added. The water preferably is acidified with a strong acid, e.g. hydrochoric acid or a similar acid to a pH value of about 3 to 4, in particular about pH 3.5. The mixture is further cooled to about 0 to 10 °C, in particular to about 5 °C and stirred at that temperature for a sustained period of time, e.g. about 1 hour. The crystalline end product is then filtered and dried.

Compound (I-a) in Form A has a particular crystalline form, i.e. the monoclinic crystalline form.

One crystalline form of compound (I-a) is referred to as 'form B' and is prepared by recrystallisation of compound (I-a) from a suitable solvent. The temperature in this recrystallisation procedure is brought above 60 °C, in particular above 70 °C. Suitable solvents are those wherein the compound of formula (I-a) dissolves at higher temperature and is relatively poorly soluble at lower temperature, e.g. at a temperature lower than 20 °C, or lower than 10 °C, or even lower than 0 °C, or -10 °C. Suitable solvents are the lower alkanols, e.g. the C₃₋₄alkanols and in particular propanol (n-propanol or 2-propanol). Other suitable solvents are esters such as ethyl acetate or a similar solvent, or mixtures thereof with lower boiling halogenated hydrocarbons such as trichloromethane or dichloromethane, which is preferred. In this instance, preferably, the starting material is first dissolved in the ethyl acetate after which the halogenated hydrocarbon is added. The volume of halogenated hydrocarbon that is used is equal to about five times (v/v) relative to the volume of ethyl acetate, preferably the volume ratio is in the range of about 1 : 2 to about 1: 5, e.g. it can be about 1 : 3 (ethylacetate : halogenated hydrocarbon). Still further suitable solvents are polyols, in particular the glycols such as ethylene glycol, which is preferred, or propylene glycol, butylene glycol and the like. Water can be added, in particular where polyols are used as a solvent. If water is added, it may be acidified with a suitable strong acid such as hydrochloric acid to low pH, e.g. a pH which is in the range of pH 2-5, e.g. a pH which is about pH 3. Water can be added in various quantities. For example where ethylene glycol or similar glycols are used, the volume ratios of glycol to added water are in the range of 1 : 1 to about 1 : 8, or 1 : 2 to 1 : 5, e.g. about 1 : 4 (glycol : water, v/v).

In one method, the compound (I-a) is dissolved by heating or refluxing in 2-propanol, ethyl acetate or a mixture of ethylacetate/dicloromethane, more preferably a 1: 3 mixture of ethylacetate/dichloromethane. Subsequently the solution is allowed to cool whereupon the desired product crystallizes.

This procedure can also be used to convert form A into form B, i.e. by replacing the compound (I-a) by form A in the above procedure.

Form B can also obtained in a highly pure form by heating form A to 130°C over a period of 60 min (DSC experiment) as triclinic crystals.

The novel crystalline forms of the compound of formula (I-a) may be characterized by their respective X-ray powder diffraction patterns utilizing appropriate powder diffractometers, in particular using the methodology as outlined in the Examples.

The following Examples describe the invention in greater detail and are intended to illustrate the invention, but not to limit it.

### Examples

### Preparation of (S)-(+)-2-(2-chlorophenyl)-2-hydroxy-ethyl carbamate (compound (I-a))

### Example 1

### (S)-(+)-2-Chloromandelic acid methyl ester (Intermediate 1)

(*S*)-(+)-2-Chloromandelic acid (100.0 g, 535.9 mmol) is dissolved at rt in methanol (553.0 g). After cooling to 10 ± 5°C, hydrogen chloride gas (55.3 g) is conducted over the solution for 30 min holding the temperature below 25 ± 5°C. The course of this reaction and the following reactions is followed by HPLC. Trimethyl orthoformate (62.6 g, 589.5 mmol) is added at 20±5°C to the colorless to light yellow solution which is stirred for 30 min at the same temperature. Thereafter the solvent and the hydrogen chloride gas is removed as far as possible *in vacuo* (40 ± 5°C, 40 ± 10 mbar). The oily residue is diluted with toluene (130 g) and the solvent is removed again as far as possible *in vacuo*. Toluene (261 g) is added and the solution is cooled to 15 ± 5°C.

### Example 2

### 2-Methoxy-1-propyl derivatized (S)-(+)-2-Chloromandelic acid methyl ester (Intermediate 2)

Hydrogen chloride gas (0.55 g, 15 mmol) is conducted over the solution (pH-value drops from 3 to 1-2). In a second flask, 2-methoxy-propene (78.0 g, 1081.7 mmol) is dissolved in toluene (120 g) at 20 ± 5°C and the above prepared solution of Intermediate 1 is added at a temperature range of 25±5°C (*ca.* 30 min). After the addition is complete, the mixture is stirred for 30 min. Triethylamine (14.0 g, 138.3 mmol) is added to the colorless to light yellow solution, it is stirred at rt for 5 min and diluted with toluene (402 g) (pH ≥ 8, if not, addition of triethylamine). The mixture is washed with water (1 x 150 g), aq. sodium hydrogencarbonate solution (5%, 1 x 150 g), and aq. sat. sodium chloride solution (1 x 150 g). Drying with sodium sulfate (*ca*. 65 g), filtration, and removal of *ca*. 86 g toluene *in vacuo* leads to Intermediate 2.

### Example 3

### 2-(2-Chlorophenyl-2-(2-(2-methoxy)propyl)-ethanol (Intermediate 3)

At first Venpure™ ERS B (13.0 g) and secondly Venpure™ ERS A (13.0 g) is added and the mixture is heated within 45 min to 85±5°C. The appearance of the solution turns from clear to slightly cloudy. Venpure™ ERS C (73.9 g, *ca*. 1239.4 mmol) is dropped into the solution in such a way that the temperature is 95 ±5° C (*ca.* 45 min, delay of the start of the exothermic reaction is possible). The reaction is finished 15 min after the end of the dropping which is also indicated by an additional milky to gray (from zinc) appearance in the mixture. If not, an additional 7.0 g of each Venpure™ ERS B and Venpure™ ERS A are added directly to the reaction mixture and the whole is heated until the conversion is complete. The solution is cooled to 15 ± 5°C and methanol (30 g) is added (hydrogen evolution). Aqueous sodium hydroxide solution (30%,238.0 g) is added dropwise while holding the temperature below 20°C (*ca.* 30 min, above 25°C, a violent foaming is observed). After the addition is fmished the two phase mixture is heated for 1 h to 50 ± 5°C, and the previously formed precipitate dissolves now. The phases are separated after cooling to rt and the colorless organic phase is washed with water (1 x 200 g) and with aq. sat. sodium chloride solution (1 x 200 g). Drying with sodium sulfate (*ca*. 65 g), filtration, and removal of *ca*. 180 g solvent *in vacuo* gives a solution of Intermediate 3.

### Example 4

### 2-(2-chlorophenyl)-2-(2-(2-methoxy)propyl)-ethyl carbamate (Intermediate 5)

To the solution obtained in example 3, a slurry of 1 ,1'-carbonyl-diimidazole (103.8 g, 641.4 mmol) in toluene (180 g) is added within 30 min. at a temperature in the range of 25±5°C. The conversion to 1-imodazolyl-1' - [2- (2-chlorophenyl) -2- [2- (2-methoxypropyl]- ethoxy carbonyl (Intermediate 4) is then complete.
Toluene (200 g) and aq. ammonium hydroxide solution (25%, 364 g) are added. After 3 h vigorous stirring at rt, the phases are separated and the organic phase containing Intermediate 5 is washed with water (1 x 200 g) and with aqueous saturated sodium chloride solution (1 x 200 g).

### Example 5

### Preparation of Compound (I-a)

To the organic phase containing Intermediate 5, prepared as described above, is added water (160 g) and cone. aq. hydrochloric acid (39 g) generating a pH-value <1.0. Compound (I-a) starts precipitating while stirring after 5 min at rt. After 4 h, the product is filtered off, the filter cake is washed with water (3 x 75 g) and with toluene (3 x 44 g). 96.9 g compound (I-a) (purity 99.8%, assay 99.0% determined by HPLC, ee >99.9% determined by chiral HPLC , 444.8 mmol, overall yield 83.0%) after drying (50±5°C, 40±10 mbar).

### Example 6

### Preparation of Form A of Compound (I-a)

This material of the previous example is suspended in methanol (115 g) and after heating to reflux, the formed solution is hot filtered, cooled to rt, and stirred for 15-20 h at this temperature. Water (577 g) containing aq. conc. hydrochloride acid (ca. 13 mg, pH-value of the solution is 3.7±0.2) is added. The mixture is cooled to 5±5°C, stirred for 2 h at the same temperature, and then filtered. The filter cake is washed with water (3x 30 g). After drying (50±5°C, 50±10 mbar), 93.0 g of Form A of compound (I-a) (purity 100%, assay 99.7%, ee >99.9%, 430.0 mmol, overall yield 80.2%) is obtained as colorless needles.

### Example 7

### Determination of polymorphic form by powder X-ray diffractometry

### Sample preparation

About 0.5 to 1 g of sample is gently pressed into a powder sample holder in order to get a well defined, smooth surface.

### Instrument

A computer controlled powder diffractometer system APD1700 (Philips) with an automated divergence slit and secondary monochromator or equivalent equipment is used.

### Recording conditions

Copper radiation: K_{α1} = 0.15406 and K_{α2} = 0.15444 nm
Voltage: 40 kV
Current: 30 mA
Divergence slit: automated
Receiving slit: 0.1°
Graphite secondary monochromator for Cu-K_{α1} Xe proportional counter

Counting starts at 1.5° 2θ and proceeds in steps of 0.02° 2θ to reach 40° 2θ. The counting time per interval is 3 sec and the total recording time is 2 h 50 min.

Form A of the compound of formula (I-a) may be characterized by its X-ray diffraction pattern, which comprises the major peaks as listed in the Table 1 in the Examples.

### TABLE 1. FORM A POWDER X-RAY DIFFRACTION PEAKS

Form B of the compound of formula (II) may be characterized by its X-ray diffraction pattern, which comprises the major peaks as listed in Table 2.

### Form A

| Angle 2-Theta ° | d value Angstrom | Intensity Cps | % |
|---|---|---|---|
| 9.217 | 9.58681 | 4610 | 100.0 |
| 10.506 | 8.41395 | 108 | 2.3 |
| 14.954 | 5.91952 | 986 | 21.4 |
| 17.375 | 5.09990 | 1501 | 32.6 |
| 18.435 | 4.80892 | 3287 | 71.3 |
| 20.028 | 4.42979 | 884 | 19.2 |
| 20.720 | 4.28343 | 286 | 6.2 |
| 21.045 | 4.21801 | 2535 | 55.0 |
| 22.205 | 4.00026 | 80.1 | 1.7 |
| 23.257 | 3.82165 | 1248 | 27.1 |
| 24.954 | 3.56544 | 494 | 10.7 |
| 25.715 | 3.46166 | 1776 | 38.5 |
| 26.093 | 3.41233 | 684 | 14.8 |
| 27.379 | 3.25489 | 3953 | 85.8 |
| 27.689 | 3.21908 | 954 | 20.7 |
| 29.339 | 3.04172 | 163 | 3.5 |
| 30.134 | 2.96333 | 517 | 11.2 |
| 31.480 | 2.83961 | 129 | 2.8 |
| 31.780 | 2.81347 | 310 | 6.7 |
| 32.139 | 2.78287 | 113 | 2.4 |
| 32.880 | 2.72180 | 318 | 6.9 |
| 33.097 | 2.70446 | 337 | 7.3 |
| 34.664 | 2.58570 | 479 | 10.4 |
| 35.170 | 2.54967 | 972 | 21.1 |
| 35.500 | 2.52667 | 464 | 10.1 |
| 35.995 | 2.49305 | 109 | 2.4 |
| 36.700 | 2.44679 | 250 | 5.4 |
| 36.958 | 2.43029 | 374 | 8.1 |
| 37.314 | 2.40791 | 1034 | 22.4 |
| 38.349 | 2.34527 | 408 | 8.9 |
| 39.222 | 2.29508 | 161 | 3.5 |
| 39.683 | 2.26945 | 310 | 6.7 |

### Example 8

### Preparation of Form B of Compound (I-a)

5.0 g (19.0 mmol) raw RWJ-333369 (polymorph A) is dissolved in 4,75 g 2-propanol by heating to reflux. This solution is cooled to room temperature within 30 min whereby the substance crystallizes. After additional stirring at 4°C for 1 h, the product is filtered off and dried at 80°C and 20 mbar for 8h. Yield: 4.0 g (15.2 mmol, 80%) polymorphic form B.

| 2-Theta | d-value | Intens. | Rel. Int. |
|---|---|---|---|
| 9.13 | 9.686 | 486.0 | 0.324 |
| 10.38 | 8.522 | 198.0 | 0.132 |
| 12.81 | 6.910 | 334.0 | 0.223 |
| 15.04 | 5.890 | 507.0 | 0.338 |
| 15.92 | 5.567 | 153.0 | 0.102 |
| 17.32 | 5.120 | 594.0 | 0.396 |
| 18.31 | 4.845 | 694.0 | 0.463 |
| 18.58 | 4.775 | 282.0 | 0.188 |
| 19.37 | 4.582 | 239.0 | 0.159 |
| 20.04 | 4.431 | 326.0 | 0.217 |
| 20.81 | 4.268 | 864.0 | 0.576 |
| 21.72 | 4.092 | 324.0 | 0.216 |
| 22.15 | 4.013 | 1500.0 | 1.000 |
| 23.25 | 3.826 | 661.0 | 0.441 |
| 24.94 | 3.570 | 416.0 | 0.277 |
| 25.64 | 3.474 | 516.0 | 0.344 |
| 27.20 | 3.278 | 1044.0 | 0.696 |
| 27.59 | 3.233 | 506.0 | 0.337 |
| 28.23 | 3.161 | 216.0 | 0.144 |
| 28.56 | 3.125 | 221.0 | 0.147 |
| 29.44 | 3.034 | 208.0 | 0.139 |
| 30.34 | 2.946 | 328.0 | 0.219 |
| 30.83 | 2.900 | 314.0 | 0.209 |
| 31.77 | 2.816 | 259.0 | 0.173 |
| 32.13 | 2.786 | 200.0 | 0.133 |
| 32.68 | 2.740 | 335.0 | 0.223 |
| 33.04 | 2.711 | 276.0 | 0.184 |
| 34.30 | 2.614 | 207.0 | 0.138 |
| 34.81 | 2.577 | 621.0 | 0.414 |
| 35.29 | 2.543 | 244.0 | 0.163 |
| 35.91 | 2.501 | 243.0 | 0.162 |
| 36.36 | 2.471 | 189.0 | 0.126 |
| 37.08 | 2.424 | 412.0 | 0.275 |
| 37.66 | 2.388 | 349.0 | 0.233 |
| 38.55 | 2.335 | 431.0 | 0.287 |
| 39.09 | 2.304 | 321.0 | 0.214 |

## Claims

1. A process for preparing a compound of formula 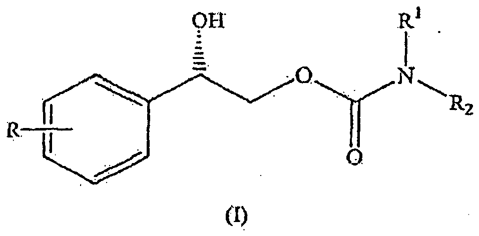 wherein:
R is halogen;
R¹ and R² independently are hydrogen or C₁₋₄alkyl, optionally substituted with phenyl or substituted phenyl, wherein substituted phenyl has substituents selected from halogen, C₁₋₄alkyl, C₁₋₄alkyloxy, amino, nitro and cyano;
**characterized by**
(a) reducing an ester of formula: 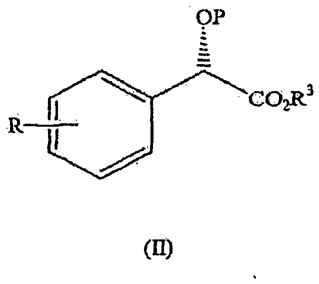 wherein:
P is an appropriate alcohol-protecting group;
R³ is C₁₋₄alkyl;
with an appropriate ester-to-alcohol reducing agent, thus obtaining an alcohol of formula: 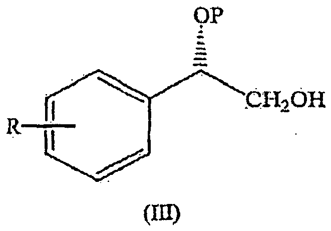
(b) reacting the alcohol of formula (III) with a carbonyl compound of formula 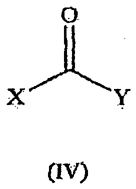 wherein X and Y are appropriate leaving groups; and subsequently with an amine of formula 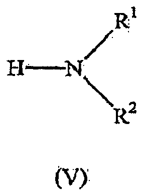 thus obtaining a compound of formula: 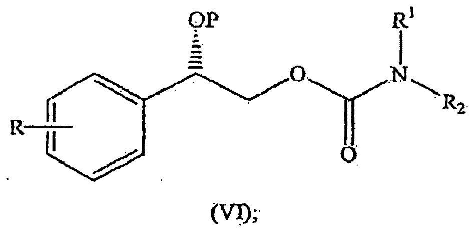
(c) removing the protecting group P thus obtaining a compound of formula(I).

2. A process according to claim 1 wherein R is 2-chloro, R¹ and R² are hydrogen.

3. A process according to claims 1 or 2 wherein the alcohol protecting group is selected from 2-(2-methoxy)propyl.

4. A process according to claims 1 or 2 wherein the carbonyl compound of formula (V) is selected from diimidazolyl carbonyl, aryl or substituted aryl haloformiate.

5. A process according to claims 1 or 2 wherein R³ is methyl.

6. A process according to claims 1 or 2 wherein the appropriate ester-to-alcohol reducing agent is a metal hydride or a complex metal hydride.

7. A compound of formula 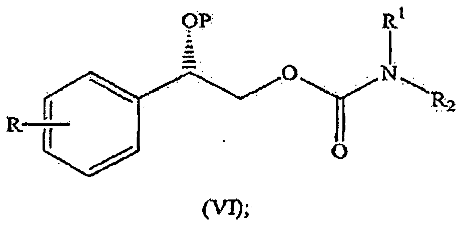 wherein R, R¹ and R² are as defined in claims 1 or 2 and P is an appropriate hydroxy-protecting group.

## Patentansprüche

1. Verfahren zur Herstellung einer Verbindung der Formel 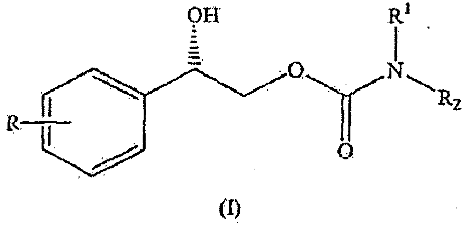 worin:
R für Halogen steht;
R¹ und R² unabhängig voneinander für Wasserstoff oder C₁₋₄-Alkyl, das gegebenenfalls durch Phenyl oder substituiertes Phenyl substituiert ist, steht, wobei das substituierte Phenyl aus Halogen, C₁₋₄-Alkyl, C₁₋₄-Alkyloxy, Amino, Nitro und Cyano ausgewählte Substituenten aufweist;
**dadurch gekennzeichnet, dass** man
(a) einen Ester der Formel: 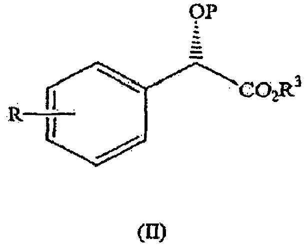 worin:
P für eine geeignete Alkohol-Schutzgruppe steht;
R³ für C₁₋₄-Alkyl steht;
mit einem geeigneten Mittel zur Reduktion von Estern zu Alkoholen reduziert, wobei man einen Alkohol der Formel: 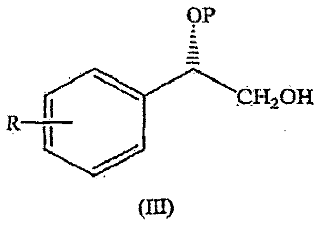 erhält;
(b) den Alkohol der Formel (III) mit einer Carbonylverbindung der Formel 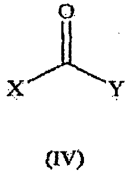 worin X und Y für geeignete Abgangsgruppen stehen; und anschließend mit einem Amin der Formel 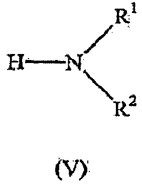 umsetzt, wobei man eine Verbindung der Formel: 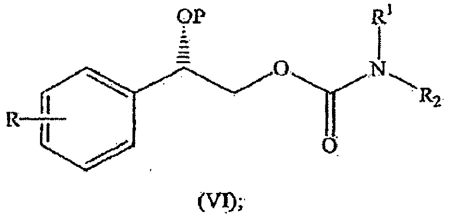 erhält;
(c) die Schutzgruppe P abspaltet, wobei man eine Verbindung der Formel (I) erhält.

2. Verfahren nach Anspruch 1, bei dem R für 2-Chlor steht und R¹ und R² für Wasserstoff stehen.

3. Verfahren nach Anspruch 1 oder 2, bei dem die Alkohol-Schutzgruppe aus 2-(2-Methoxy)propyl ausgewählt wird.

4. Verfahren nach Anspruch 1 oder 2, bei dem die Carbonylverbindung der Formel (V) aus Diimidazolylcarbonyl und Aryl- oder substituiertem Arylhalogenformiat ausgewählt ist.

5. Verfahren nach Anspruch 1 oder 2, bei dem R³ für Methyl steht.

6. Verfahren nach Anspruch 1 oder 2, bei dem es sich bei dem geeigneten Mittel zur Reduktion von Estern zu Alkoholen um ein Metallhydrid oder ein komplexes Metallhydrid handelt.

7. Verbindung der Formel 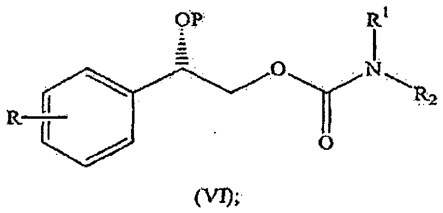 worin R, R¹ und R² wie in Anspruch 1 oder 2 definiert sind und P für eine geeignete HydroxySchutzgruppe steht.

## Revendications

1. Procédé de synthèse d'un composé de formule 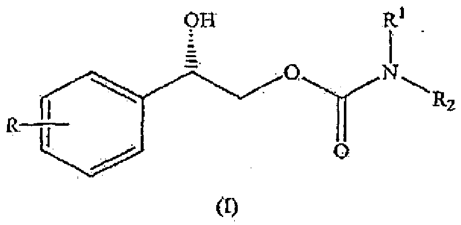 où :
R représente un atome d'halogène ;
chacun des radicaux R¹ et R² représente indépendamment un atome d'hydrogène ou un groupement alkyle en C₁₋₄,
éventuellement substitué par phényle ou phényle substitué, où le groupement phényle substitué porte des substituants choisis parmi les atomes d'halogène et les groupements alkyle en C₁₋₄, (alkyle en C₁₋₄)-oxy, amino, nitro et cyano ;
**caractérisé par**
(a) la réduction d'un ester de formule : 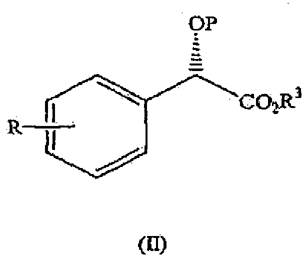 où :
P représente un groupement protecteur de fonction alcool adapté ;
R³ représente un groupement alkyle en C₁₋₄ ;
avec un agent réducteur d'ester en alcool adapté, ce qui permet d'obtenir un alcool de formule : 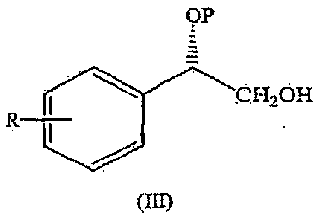
(b) la réaction de l'alcool de formule (III) avec un carbonyle de formule 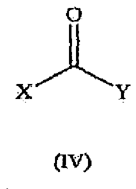 où X et Y représentent des groupements partants adaptés ; puis avec une amine de formule 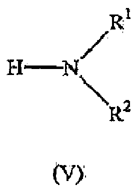 ce qui permet d'obtenir un composé de formule : 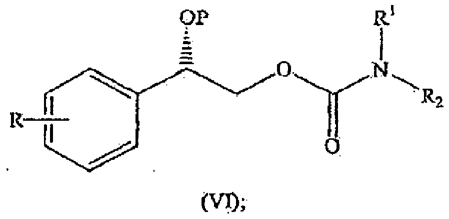
(c) le clivage du groupement protecteur P, pour obtenir un composé de formule (I).

2. Procédé selon la revendication 1, où R représente un groupement 2-chloro, chacun des radicaux R¹ et R² représente un atome d'hydrogène.

3. Procédé selon les revendications 1 ou 2, où le groupement protecteur de fonction alcool est choisi parmi les groupements 2-(2-méthoxy)propyle.

4. Procédé selon les revendications 1 ou 2, où le carbonyle de formule (V) est choisi parmi les groupements diimidazolylcarbonyle, aryle ou halogénoformiate d'aryle substitué.

5. Procédé selon la revendication 1 ou 2, où R³ représente un groupement méthyle.

6. Procédé selon les revendications 1 ou 2, où l'agent réducteur d'ester en alcool adapté est un hydrure métallique ou un complexe d'hydrure métallique.

7. Composé de formule 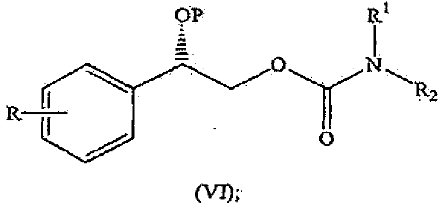 où R, R¹ et R² sont tels que définis dans les revendications 1 ou 2 et P représente un groupement protecteur de fonction hydroxy adapté.
